# EUROPEAN PATENT APPLICATION

(11) **EP 2 581 084 A1**
(43) Date of publication of application: **17.04.2013**
(21) Application number: 11789700.9
(22) Date of filing: 27.05.2011
(51) Int. Cl.: A61K 31/4196, A61K 9/14, A61K 9/16, A61K 9/20, A61K 9/48, A61K 47/32, A61K 47/38, A61P 7/02, A61P 25/04, A61P 29/00, A61P 35/00, A61P 37/02, A61P 37/06

(54) **SOLID DISPERSION COMPRISING TRIAZOLE COMPOUND**

(30) Priority: 31.05.2010 JP 2010124292
(71) Applicant: Astellas Pharma Inc., Tokyo 103-8411 (JP)
(72) Inventor: SAKURAI, Atsushi, Tokyo 103-8411 (JP); SAKO, Kazuhiro, Tokyo 103-8411 (JP); SAKAI, Toshiro, Tokyo 103-8411 (JP)
(74) Representative: Marshall, Cameron John
(86) International application number: PCT/JP2011/062172
(87) International publication number: WO 2011/152297

(57) **Abstract**

A solid dispersion comprising a poorly soluble pharmaceutical compound, such as 3-methoxy-1,5-bis(4-methoxyphenyl)- 1H-1,2,4-triazole, which has excellent ease of handling and stability is provided. The solid dispersion comprises a polymeric carrier (for example, a polymer selected from a group consisting of polyvinylpyrrolidone and copolyvidone) and further comprising hydroxypropyl methylcellulose as desired.

## Description

### Technical Field

The present invention concerns solid dispersions which include triazole compounds such as 3-methoxy-1,5-bis(4-methoxyphenyl)-1H-1,2,4-triazole (referred to hereinafter as "Compound A") and the like. More precisely the invention concerns solid dispersions which include Compound A (or other triazole compound) and a polymeric carrier which are amorphous and of which the glass transition point is 40°C or above. Furthermore, the present invention concerns pharmaceutical compositions which include said solid dispersions, methods for the production of said solid dispersions and methods for the treatment disease in humans in which said pharmaceutical compositions are used.

### Technical Background

Triazole compounds, including 3-methoxy-1,5-bis(4-methoxyphenyl)-1H-1,2,4-triazole (referred to hereinafter as "Compound A"), which have cyclooxygenase inhibitory activity have been described in Japanese Domestic Patent (Kohyo) 2004-521964. Compound A is useful as a pharmaceutical compound due to its inhibitory activity, but at the same time its solubility in a variety of solvents is low. Furthermore, see also PCT Publication Nos. WO 03/040110 and WO 09/123210, U.S. Patent No. 6,927,230 and U.S. Laid Open Patent Application Nos. 2008-0213383 and 2011-0034504 (all of which are incorporated herein by reference).

Solid dispersions have been prepared from sparingly soluble pharmaceutical compounds and water soluble polymeric carriers by means of solvent methods, melting methods, pulverizing methods and the like as a way to improve solubility and absorption. A solid dispersion is generally a dispersion where an active component (compound) has been dissolved or dispersed in the solid state in an inert carrier (matrix) such as a water soluble polymer.

For example, a solid dispersion which has been produced with a twin-screw extruder has been described in WO 92/181606 with a view to resolving the problems associated with producing solid dispersions with a single-screw extruder or by means of a solvent method. A solid dispersion in which any two types of water soluble polymer selected from among the group comprising hydroxypropyl methylcellulose, hydroxypropyl cellulose and polyvinylpyrrolidone are used for the carrier has been described in WO 01/95941.

### Prior Art Literature

### Patent Documents

Patent Document 1:
   Japanese Domestic Patent (Kohyo) 2004-521964
Patent Document 2:
   WO 03/040110
Patent Document 3:
   WO 09/123210
Patent Document 4:
   U.S. Patent No. 6,927,230
Patent Document 5:
   Laid Open U.S. Patent Application 2008-0213383
Patent Document 6:
   Laid Open U.S. Patent Application 2011-0034504
Patent Document 7:
   WO 92/18106
Patent Document 8:
   WO 01/95941

### Outline of Invention

### Problems to be Resolved by the Invention

3-Methoxy-1,5-bis(4-methoxyphenyl)-1H-1,2,4-triazole (Compound A) is useful as a pharmaceutical compound but, because it has low solubility in a variety of solvents, it is difficult to form into a pharmaceutical preparations. Other triazole compounds (including but not limited to those described in U.S. Patent No. 6,927,230 for example) also have similar low solubility and improved pharmaceutical preparations are required for such compounds.

Attempts to improve the bioavailability of sparingly soluble pharmaceutical compounds have been made by forming solid dispersions in the way described above. However, satisfactory dispersions of Compound A cannot always be prepared with the existing methods for the production of solid dispersions because of the properties of the compound.

When the inventors produced a solid dispersion of Compound A by means of the melting method using hydroxypropyl methylcellulose (referred to hereinafter as "HPMC") which is a typical polymer used in solid dispersions or the like it was found that the solid dispersion obtained had a rubber-like form at room temperature and was therefore very difficult to pulverize (the measured glass transition point of this solid dispersion was 12°C).

From the viewpoint of proper solubility a pharmaceutical compound is preferably present in a solid dispersion in an amorphous form which is stable with the passage of time. However, in solid dispersions produced from Compound A and HPMC a large amount of the Compound A which was originally in an amorphous form is transformed during storage into a crystalline form and there is a problem with the stability of the amorphous form. It has been conjectured that such a problem may be the cause of the poor solubility of Compound A in water and organic solvents and the low glass transition temperature of solid dispersions which contain Compound A.

Hence, the aim of the present invention is to provide novel solid dispersions which include Compound A or other similar sparingly soluble triazole compound (including but not limited to, for example, those described in U.S. Patent No. 6,927,230 (incorporated in this specification by reference)), which have excellent handlability and stability.

### Means of Resolving These Problems

The present invention arose in part from the discovery that solid dispersions of Compound A that are easily handled as preparations can be obtained by using polymers selected from the group consisting of polyvinylpyrrolidone (referred to hereinafter as "PVP") and copolyvidone and moreover that by using hydroxypropyl methylcellulose conjointly solid dispersions which exhibit raised solubility and super-saturation retention and which are also excellent in terms of bioavailability can be obtained.

That is to say, the present invention is:
[1] A solid dispersion which includes a triazole compound which can be represented by formula (I):
   [Ka-1] 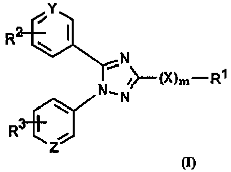 [In this formula R¹ is a lower alkyl group optionally substituted with a halogen, cyano, N,N-di(lower)alkylcarbamoyl, phenyl which may be optionally substituted with halogen, or heterocyclic group,
   a cyclo(lower)alkyl group,
   a lower alkynyl group or
   an N,N-di(lower)alkylcarbamoyl group;
   R² is a lower alkyl group, lower alkoxy group, cyano group or 1H-pyrrol-1-yl group;
   R³ is a lower alkyl group, lower alkoxy group or cyano group;
   X is O, S, SO or SO₂;
   Y and Z are each CH or N;
   m is 0 or 1];
   and a polymeric carrier, the solid dispersion being amorphous and having a glass transition point of 40°C or above (for example 80°C),
[2] The solid dispersion described in [1] wherein the triazole compound is 3-methoxy-1,5-bis(4-methoxyphenyl)-1H-1,2,4-triazole,
[3] The solid dispersion described in [1] or [2] in which the polymeric carrier is of one type or of two types selected from the group comprising polyvinylpyrrolidone and copolyvidone,
[4] The solid dispersion described in any one of [1] to [3] which also includes hydroxypropyl methylcellulose,
[5] The solid dispersion described in [4] in which hydroxypropyl methylcellulose is compounded in the polymeric carrier,
[6] The solid dispersion described in [1] in which the amount of polymeric carrier included per 1 part by mass of triazole compound represented by formula (I) is from 1 to 10 parts by mass,
[7] The solid dispersion described in [4] or [5] in which the amount of hydroxypropyl methylcellulose included per 1 part by mass of triazole compound represented by formula (I) is from 1 to 3 parts by mass,
[8] The solid dispersion described in any one of [1] to [7] which has been produced by means of a melting method,
[9] A pharmaceutical composition which includes the solid dispersion described in any one of claims 1 to 8, and
[10] A method for producing the solid dispersion described in [1] which includes:
   a process in which a triazole compound represented by formula (I) and a polymeric carrier are mixed and a mixture is prepared; and
   a process in which said mixture is melted.

### Effect of the Invention

It is possible by means of the present invention to provide solid dispersions which have improved handlability and stability (retention of the amorphous form).

### Brief Description of Drawings

Figure 1:

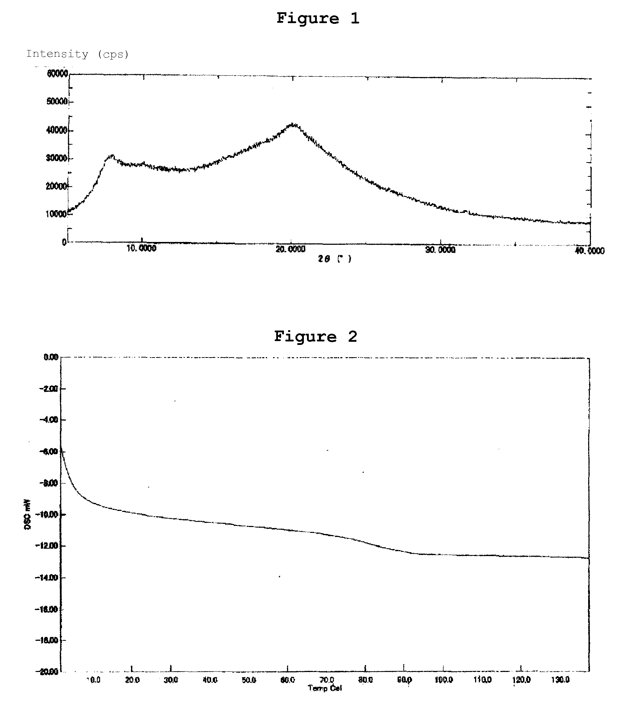

   Figure 1 is an X-ray diffraction measurement diagram of the solid dispersion of Example 7 after storage.
Figure 2:
   Figure 2 is a differential scanning calorimetric measurement diagram of the solid dispersion of Example 7 after storage.

### Embodiment of the Invention

In this specification "excellent handlability" signifies a state where, for example, the solid dispersion is easily processed when being pulverized.

In this specification "excellent stability" signifies that when, for example, particles of the solid dispersion are taken in a plastic container and stored for 1 month under conditions of 40°C and humidity 75% and the crystallinity of the triazole compound which can be represented by formula (I) (for example Compound A) which is included in the dispersion is evaluated using powder X-ray diffraction measurements (RINT TTRIII, produced by the Rigagaku Denki Co.) a broad pattern with no diffraction peaks (halo pattern) is confirmed.

In this specification "easily pulverized" signifies that, for example, it is possible to pulverize a solid dispersion after melt milling. In another way it signifies a state where the production yield after pulverizing a solid dispersion is at least 80 mass% with respect to the mass before pulverization.

In this specification "difficult to pulverize" signifies a state in which, for example, the solid dispersion cannot be pulverized after melt milling and the recovery of solid dispersion after pulverization is low. In another way it signifies a state where the production yield after pulverizing a solid dispersion is not more than 20 mass% with respect to the weight before pulverization.

A solid dispersion of this invention is a solid dispersion which includes a triazole compound represented by formula (I):
[Ka-2] 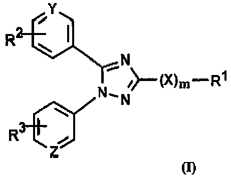 [In this formula R¹ is a lower alkyl group optionally substituted with a halogen, cyano, N,N-di(lower)alkylcarbamoyl, phenyl optionally substituted with halogen, or heterocyclic group,
a cyclo(lower)alkyl group,
a lower alkynyl group, or
an N,N-di(lower)alkylcarbamoyl group;
R² is a lower alkyl group, lower alkoxy group, cyano group or 1H-pyrrol-1-yl group;
R³ is a lower alkyl group, lower alkoxy group or cyano group;
X is O, S, SO or SO₂;
Y and Z are each CH or N;
m is 0 or 1];
and a polymeric carrier, the solid dispersion being amorphous and having a glass transition point of 40°C or above.

Here the term "lower" has no specific limits but signifies a group which has from 1 to 6 carbon atoms.

The ideal lower alkyl parts for a "lower alkyl group" and "lower alkoxy" include those which have linear chains or which are branched, and examples include methyl, ethyl, propyl, isopropyl, butyl, isobutyl, tertiary butyl, pentyl, hexyl and the like, and methyl or ethyl can be cited as preferred examples.

Examples of ideal lower alkoxy groups include methoxy, ethoxy, propoxy, isopropoxy, butoxy, isobutoxy, tertiary butoxy, pentoxy, hexoxy and the like, and of these methoxy is preferred.

Fluorine, chlorine, bromine, iodine and the like can be cited as ideal "halogens", and of these fluorine is preferred.

The ideal "lower alkyl groups substituted with halogen" are lower alkyl substituted with one or more halogen atom, and examples include fluoromethyl, difluoromethyl, trifluoromethyl, chloromethyl, dichloromethyl, trichloromethyl, bromomethyl, dibromomethyl, tribromomethyl, fluoroethyl, chloroethyl, 2,2,2-trifluoroethyl, 2,2,2-trichloroethyl, 2,2,3,3,3-pentafluoroethyl, fluoropropyl, fluorobutyl, fluorohexyl and the like. Of these a C1 or C2 alkyl which has been substituted with halogen is preferred. From among these trifluoromethyl or 2,2,2-trifluoroethyl is the most desirable.

The ideal "cyclo (lower) alkyl groups" are three to eight membered cycloalkyl, and examples include cyclopropyl, cyclobutyl, cyclopentyl, cyclohexyl, cycloheptyl, cyclo-octyl and the like, and those which have from five to seven carbon atoms are preferred.

The ideal "N,N-di(lower)alkylcarbamoyl groups" are carbamoyl groups which have been substituted on the nitrogen atom with the aforementioned alkyl groups which may be the same or different, and examples include dimethylcarbamoyl, diethylcarbamoyl, dipropylcarbamoyl, diisopropylcarbamoyl and the like. From among these a di(C1 to C4 alkyl)carbamoyl is preferred and a di(C1 or C2 alkyl)carbamoyl is more desirable.

The ideal "heterocyclic groups" are saturated or unsaturated single-ring or multi-ring heterocyclic groups which have at least one hetero-atom such as an oxygen atom, sulfur atom, nitrogen atom or the like. Especially desirable heterocyclic groups include, for example, the three to eight membered (and preferably five or six membered) unsaturated heterocyclic single-ring groups which have from one to four nitrogen atoms, for example pyrrolyl, pyrrolinyl, imidazolyl, pyrazolyl, pyridyl and its N-oxide, dihydropyridyl, pyrimidyl, pyrazinyl, pyridazinyl, tetrahydro-pyridazinyl, (for example 2,3,4,5-tertrahydro-pyridazinyl and the like), triazolyl (for example 4H-1,2,4-triazolyl, 1H-1,2,3-triazolyl, 2H-1,2,3-triazolyl and the like), tetrazolyl (for example 1H-tetrazolyl, 2H-tetrazolyl and the like) and the like; the three to eight membered (and preferably five or six membered) saturated single-ring heterocyclic groups which have from one to four nitrogen atoms, for example pyrrolidinyl, imidazolidinyl, piperidino, piperazinyl and the like; the unsaturated condensed heterocyclic groups which have from one to four nitrogen atoms, for example indolyl, isoindolyl, indolinyl, isoindolinyl, benzimidazolyl, quinolyl, isoquinolyl, indazolyl, benzotriazolyl and the like: the three to eight membered (and preferably five or six membered) unsaturated single-ring heterocyclic groups which have one or two oxygen atoms and from one to three nitrogen atoms, for example oxazolyl, iso-oxazolyl (for example 3-iso-oxazolyl), oxadiazolyl and the like (for example 1,2,4-oxadiazolyl, 1,3,4-oxadiazolyl, 1,2,5-oxadiazolyl and the like) and the like; the three to eight membered (and preferably five or six membered) saturated single-ring heterocyclic groups which have one or two oxygen atoms and from one to three nitrogen atoms, for example morpholinyl, sydononyl and the like; the unsaturated condensed heterocyclic groups which have one or two oxygen atoms and from one to thee nitrogen atoms, for example benzo-oxazolyl, benzo-oxadiazolyl and the like; the three to eight membered (and preferably five or six membered) unsaturated single-ring heterocyclic groups which have one or two sulfur atoms and from one to three nitrogen atoms, for example thiazolyl (for example 1,3-thiazolyl), isothiazolyl, thiadiazolyl (for example 1,2,3-thiadiazolyl, 1,2,4-thiadiazolyl, 1,3,4-thiadiazolyl, 1,2,5-thiadiazolyl and the like), dihydrothiazinyl and the like; the three to eight membered (and preferably five or six membered) saturated single-ring heterocyclic groups which have one or two sulfur atoms and from one to thee nitrogen atoms, for example thiazolidinyl and the like; the three to eight membered (and preferably five or six membered) unsaturated single-ring heterocyclic groups which have one or two sulfur atoms, for example thienyl, dihydrodithienyl, dihydrodithionyl and the like; the unsaturated condensed heterocyclic groups which have one or two sulfur atoms and from one to three nitrogen atoms, for example, benzothiazolyl, benzothiadiazolyl and the like; the three to eight membered (and preferably five or six membered) unsaturated single-ring heterocyclic groups which have one oxygen atom, for example furyl and the like; the three to eight membered (and preferably five or six membered) unsaturated single-ring heterocyclic groups which have one oxygen atom and one or two sulfur atoms, for example, dihydro-oxathienyl and the like; the unsaturated condensed heterocyclic groups which have one or two sulfur atoms, for example benzothienyl, benzodithienyl and the like; and the unsaturated condensed heterocyclic groups which have one oxygen atom and one or two sulfur atoms, for example benzo-oxathienyl and the like; and the like.

A halogen, such as chlorine for example, can be cited as an ideal "leaving group".

The monovalent branched or unbranched hydrocarbon radicals, for example ethynyl, 2-propynyl, 2-butynyl and the like, can be cited as ideal alkynyl groups.

CH and CH, CH and N or N and CH can be cited as the preferred combinations of Y and Z.

The compounds described in U.S. Patent No. 6,927,230, for example,
1,5-bis(4-methoxyphenyl)-3-trifluoromethyl)-1H-1,2,4-triazole,
1,5-bis(4-methoxyphenyl)-3-trifluoromethyl)-1H-1,2,4-triazole,
4-[1-(4-methoxyphenyl)-3-(trifluoromethyl)(-1H-1,2,4-triazol-5-yl]benzonitrile, 1-(4-methoxyphenyl)-5-(4-methylphenyl)-2-(trifluoromethyl)-1H-1,2,4-triazole,
3-methoxy-1,5-bis(4-metghoxyphenyl)-1H-1,2,4-triazole, 1,5-bis(4-methoxyphenyl)-3-(methylthio)-1H-1,2,4-triazole,
1,5-bis(4-methoxyphenyl)-3-(methylsulfinyl)-1H-1,2,4-triazole,
1,5-bis(4-methoxyphenyl)-3-(methylsulfonyl)-1H-1,2,4-triazole,
1-(4-methoxyphenyl)-5-[4-(1H-pyrrol-1-yl)phenyl]-3-(trifluoromethyl)-1H-1,2,4-triazole,
2-methoxy-5-[1-(4-methoxyphenyl)-3-(trifluoromethyl)-1H-1,2,4-triazol-5-yl]pyridine,
4-[5-(4-methoxyphenyl)-3-(trifluoromethyl)-1H-1,2,4-triazol-1-yl]benzonitrile ,
3-ethoxy-1,5-bis(4-methoxyphenyl)-1H-1,2,4-triazole, 5-(4-ethoxyphenyl)-1-(4-methgylphenyl)-3-(trifluoromethyl)-1H-1,2,4-triazole,
2-methoxy-5-[5-(4-methoxyphenyl)-3-(trifluoromethyl)-1H-1,2,4-triazol-1-yl]pyridine,
1,5-bis(4-methoxyphenyl)-3-(2,2,2-trifluoroethoxy)-1H-1,2,4-triazole,
2-methoxy-5-(1-(4-methoxyphenyl)-3-(2,2,2-trifluoro-ethoxy)-1H-1,2,4-triazol-5-yl)pyridine,
2-methoxy-5-(5-(4-methoxyphenyl)-3-(2,2,2-trifluoro-ethoxy)-1H-1,2,4-triazol-1-yl)pyridine, 1,5-bis(4-methoxyphenyl)-1H-1,2,4-triazol-3-yl-dimethylcabamamic acid salt,
1,5-bis(4-methoxyphenyl)-3-(2-propynyloxy)-1H-1,2,4-triazole,
3-({[1,5-bis(4-methoxyphenyl)-1H-1,2,4-triazol-3-yl]oxy}methyl)-1,2,4-oxaziazole,
1,5-bis(4-methoxyphenyl)-3-[(5-methyl-3-iso-oxazolyl)-methoxy]-1H-1,2,4-triazole,
1,5-bis(4-methoxyphenyl)-3-(1,3-thiazol-4-ylmethoxy)-1H-1,2,4-triazole,
2-{[1,5-bis(4-methoxyphenyl)-1H-1,2,4-triazol-3-yl]oxy}-N,N-dimethylacetamide,
1,5-bis(4-methoxyphenyl)-3-(2-butynyloxy)-1H-1,2,4-triaxzole,
1,5-bis(4-methoxyphenyl)-3-(2-propoxy)-1H-1,2,4-triazole,
1,5-bis(4-methoxyphenyl)-3-(fluoromethoxy)-1H-1,2,4-triazole,
1,5-bis(4-methoxyphenyl)-3-cyclohexyloxy-1H-1,2,4-triazole,
1,5-bis(4-methoxyphenyl)-3-(4-chlorophenylmethoxy)-1H-1,2,4-triazole, and
1,5-bis(4-methoxyphenyl)-3-cyanomethoxy-1H-1,2,4-triazole can be cited as compounds of formula (I).

In one embodiment of the invention the solid dispersion is an amorphous solid dispersion of glass transition point 40°C or above which includes 3-methoxy-1,5-bis(4-methoxyphenyl)-1H-1,2,4-triazole (Compound A) and a polymeric carrier.

Compound (A) is a triazole compound which has the structure of formula (II).
[Ka-3] 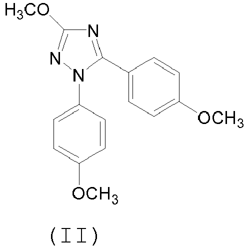 This compound can be produced by means of the method described in Example 5 of the Japanese Domestic Patent (Kohyo) 2004-521964 (Example 5 of U.S. Patent 6,927,230). Furthermore, Compound A has the following properties: glass transition point 4°C, solubility in water (37°C) 10.7 µg/mL and solubility in organic solvent [acetone: 82.2 mg/mL, ethanol: 15.9 mg/mL and polyethylene glycol: 6.3 mg/mL (all at room temperature)].

No particular limitation is imposed upon the polymeric carrier which is used in the present invention provided that it is a substance which is pharmacologically acceptable, can form a solid dispersion having a glass transition point of 40°C or above with a triazole compound which can be represented by formula (I) (for example Compound A) and with which pharmacologically acceptable solid dispersions of triazole compounds which can be represented by formula (I) can produced at normal temperature.

Examples include polyvinylpyrrolidone (PVP) and copolyvidone. One type, or two or more types, of said polymeric carrier can be used in an appropriate amount for said polymeric carrier.

Polyvinylpyrrolidone is a homopolymer of vinylpyrrolidone. No particular limitation is imposed upon the PVP provided that it one which can be used in field of pharmaceutical preparations and, for example, those of weight average molecular weight some 40,000 to 360,000 can be used. In more practical terms, for example, a commercially available PVP such as those with the trade names PVP K30 or PVP K90 and the like can be used.

Copolyvidone is a copolymer of 1-vinyl-2-pyrrolidone and vinyl acetate with a mass ratio of 3:2. No particular limitation is imposed upon the copolyvidone provided that it can be used in the field of pharmaceutical preparations. In more practical terms, for example, the commercially available copolyvidones such as those with the trade names Kollidon VA64 (produced by the BASF Co.) and Plasdone S630 (produced by the ISP Co.) can be used.

The proportion in which the polymeric carrier from which the solid dispersion of the present invention made is made is compounded is from 1 to 10 parts by mass, and preferably from 2 to 5 parts by mass, per 1 part by mass of the triazole compound which can be represented by formula (I). A combination of PVP and copolyvidone can also be used in a solid dispersion of the present invention. In this case they are used in amounts such that the total mass of PVP and copolyvidone has the value indicated above, and the proportions of PVP and copolyvidone can be adjusted appropriately.

In those cases where the proportion of polymeric carrier compounded is below the abovementioned value the glass transition point of the solid dispersion may be reduced and, furthermore, handling of the solid dispersion becomes difficult. In those cases where the polymeric carrier is compounded in a proportion greater than the abovementioned value the proportion of the compound which can be represented by formula (I) in the solid dispersion is reduced and so the mass of the drug required to administer the effective dose of the triazole compound which can be represented by general formula (I) is increased.

A solid dispersion comprising a triazole compound which can be represented by formula (I) and a polymeric carrier can also include hydroxypropyl methylcellulose (HPMC). The solubility and super-saturation in water of the solid dispersion, for example, can be improved by using HPMC.

Hydroxypropyl methylcellulose is a cellulose ether where hydroxypropoxyl group has been introduced into methylcellulose. No particular limitation is imposed upon the HPMC provided that is can be used in the field of pharmaceutical preparations. For example, material which has a hydroxypropoxyl group content of some 7 to 12% can be used. In more practical terms, for example, the commercially available HPMC with the trade names TC-5E and TC-5R (manufactured by Shin-Etsu Chemical Co.) can be used.

The proportion of HPMC compounded is, for example, from 1 to 3 parts by mass per 1 part by mass of the triazole compound which can be represented by formula (I). In those cases where the proportion of HPMC compounded is below than the abovementioned value there is concern that the dissolving out properties may be unsatisfactory and that the state of super-saturation of the compound which can be represented by formula (I) may not be retained satisfactorily.

A solid dispersion of the present invention can include other optional components which can be used in pharmaceutical preparations as well and the components indicated above. Excipients, binding agents, stabilizers, disintegrants, acidulants, foaming agents, artificial sweeteners, flavors, lubricants, coloring agents, buffering agents, antioxidants, surfactants, fluidizers, coating agents and the like can be cited as examples of these other components.

For example, lactose, crystalline cellulose, microcrystalline cellulose, D-sorbitol, D-mannitol, xylitol, compressible sugar, corn starch, potato starch, wheat starch, rice starch, calcium carbonate, calcium sulfate, monobasic calcium phosphate, dibasic calcium phosphate (anhydrous and dihydrate), tribasic calcium phosphate, dibasic sodium phosphate, dibasic potassium phosphate, magnesium oxide, magnesium carbonate and the like can be cited as excipients.

Examples of the binding agents include gum arabic, hydroxypropyl cellulose and derivatives thereof, hydroxyethylcellulose, polyethylene glycol, methacrylic acid copolymers and derivatives thereof, polyvinyl alcohol and the like.

Examples of the stabilizers include yellow iron sesquioxide, red iron sesquioxide, black ferric oxide and the like.

Examples of the disintegrants include corn starch, potato starch, carmellose calcium, carmellose sodium, low-substituted hydroxypropyl cellulose and the like.

Examples of the acidulants include citric acid, tartaric acid, malic acid and the like.

Examples of the foaming agents include sodium bicarbonate and the like.

Examples of the artificial sweeteners include saccharin sodium, dipotassium glycyrrhizinate, aspartame, stevia, somatin and the like.

Examples of the flavors include lemon, lemon-lime, orange, menthol and the like.

Examples of the lubricants include magnesium stearate, calcium stearate, sucrose fatty acid esters, polyethylene glycol, talc, stearic acid and the like.

Examples of the coloring agents include food yellow Nos. 4 and 5, food red Nos. 3 and 102, food blue No. 3 and the like.

Examples of the buffering agents include citric acid, succinic acid, fumaric acid, tartaric acid, ascorbic acid or salts thereof, glutamic acid, glutamine, glycine, aspartic acid, alanine, arginine or salts thereof, magnesium oxide, zinc oxide, magnesium hydroxide, phosphoric acid, boric acid and salts thereof and the like.

Examples of the antioxidants include ascorbic acid, dibutylhydroxytoluene, propyl gallate and the like.

Examples of the surfactants include polysorbate 80, sodium lauryl sulfate, polyoxyethylene hardened castor oil and the like.

Examples of the fluidizers include silicon dioxide, aluminum silicate, magnesium aluminosilicate, calcium triphosphate and the like.

Examples of the coating agents include hydroxypropyl methylcellulose, HPC, povidone, polyvinyl alcohol, methylcellulose, gum arabic, polyethylene glycol, copolyvidone, gelatin, dextrin, methacrylic acid copolymers, ethyl acrylate-methacrylic acid copolymers, aminoalkyl methacrylate copolymer E, aminoalkyl methacryl copolymer RS, ethyl cellulose, carboxyvinyl polymer, carboxymethylethyl cellulose, carboxymethyl starch sodium, carmellose calcium, carmellose sodium and the like.

One type, or a combination of two or more types, of the above components can be compounded in an appropriate amount. In terms of the amounts compounded for all of the excipients an amount within a range where the pharmaceutical preparation obtained, in a practical pharmaceutical application, has the desired effect of the present invention can be used.

A solid dispersion of the present invention can be produced by mixing the polymeric carrier, the triazole compound which can be represented by formula (I) and also HPMC and the abovementioned optional components as required and then melting, cooling and solidifying the mixture (melting process). The melting can be carried out at a temperature of, for example, 120 to 200°C. Furthermore, following the melting, cooling and solidification, processes such as pulverization and the like can be carried out as required.

The melting method is a method with which a solid dispersion is obtained by subjecting the raw drug and the polymeric carrier to a heat treatment or heat and milling treatment and can be sub-divided into the static melting method and the melt-milling method. The static melting method is a method for obtaining a solid dispersion by subjecting the pre-mixed raw drug and polymeric carrier, or raw drug, polymeric carrier and the various additives, (referred to as the mixture) to a heat treatment using a circulating hot air oven or the like. The melt-milling method is a method for obtaining a solid dispersion by subjecting the mixture to a heating and milling treatment using a single-screw extruder or a multi-screw extruder. An appropriate amount of solvent, plasticizer or the like can be added when subjecting the mixture to the heating and milling treatment.

For example, a solid dispersion of the present invention can be produced in the way outlined below using a heater-equipped agitation granulating machine.

First of all a physical mixture of the polymeric carrier, triazole compound which can be represented by formula (I), and also HPMC and the above optional ingredients which can be used as required, is prepared beforehand. Then the mixture is granulated using a heater-equipped agitation granulating machine. The operating conditions such as the treatment temperature and the treatment time for example differ according to the types and compounding proportions of the polymeric carrier and the optional components which are being used, but the treatment temperature is, for example, from room temperature (about 20°C) to 200°C and the treatment time is, for example, from 5 minutes to 20 hours. Then a solid dispersion is obtained by cooling. The temperature for cooling is preferably from -80°C to room temperature.

Furthermore, a solid dispersion of the present invention can be produced by means of the hot press melt-milling method. In the hot press melt-milling method the triazole compound which can be represented by formula (I) and the polymeric carrier are mixed while applying heat and pressure. The operating conditions are such as the treatment temperature, treatment pressure and treatment time, for example, differ according to the types and compounding proportions of the polymeric carrier and the optional components which are being used, but the treatment temperature is from room temperature to 200°C, the treatment pressure is from 1 to 20 MPa and the treatment time is from 5 minutes to 20 hours. A solid dispersion which has been produced by means of a hot press melt-milling method can be produced, for example, using a closed type continuous twin-screw milling machine (kneader), a twin-screw extruder or the like which has been equipped with a heater. In more practical terms a solid dispersion can be produced in the way outlined below.

The triazole compound which can be represented by formula (I), the polymeric carrier, and the HPMC and abovementioned optional components which can be used as required, are physically mixed beforehand. The resulting mixture is supplied to a closed continuous twin-screw milling machine at a powder supply rate of from 3 to 300 g/min. The treatment is carried out with a screw rotation rate of from 10 to 300 rpm and a barrel temperature of from 40 to 200°C. A glass-like solid dispersion is obtained by this means. A solid dispersion powder is then obtained by pulverization in a pulverizing machine. Moreover, pressure due to milling is applied to the triazole compound which can be represented by formula (I), polymeric carrier etc. which have been supplied inside the milling machine.

In a solid dispersion of the present invention which has been produced by means of a melting method the triazole compound which can be represented by the formula (I) is uniformly dissolved or dispersed in an amorphous state in a matrix of the polymeric carrier (or a matrix which includes the polymeric carrier and HPMC in those cases where HPMC has been used).

A solid dispersion of the present invention is amorphous and its glass transition point is 40°C or above (and preferably 50°C or above, for example from 50 to 90°C, or from 60 to 80°C). Consequently with a solid dispersion of the present invention the amorphous state of the triazole compound which can be represented by formula (I) can be maintained in a stable manner over a long period of time. Moreover, a solid dispersion of the present invention can be easily pulverized and this is excellent from the handling viewpoint.

Whether the triazole compound which can be represented by formula (I) in a solid dispersion is in an amorphous or crystalline form can be ascertained by means of differential scanning calorimetric measurements (DSC) or X-ray diffraction measurements. With X-ray diffraction measurements a sharp diffraction peak pattern is obtained in the case of a crystalline form and a broad pattern (halo pattern) without any clear diffraction peaks is obtained in the case of an amorphous form. With DSC a peak is observed in the case of a crystalline form but no peak is observed in the case of an amorphous form.

The glass transition point of a solid dispersion can be obtained by subjecting the solid dispersion to calorimetric measurements using DSC and plotting a curve of changes in the amount of heat. In more practical terms a sample of about 10 mg is weighed out onto an aluminum pan and calorimetry is carried out for temperatures from 0°C and 150°C with a rate of temperature increase of 5°C per minute. In the process of heating the sample heat is absorbed or generated at the glass transition point and, as a result, the baseline of the calorimetric curve will shift downward. Extended lines are drawn for the two baselines, namely the original baseline and the shifted baseline, and the glass transition temperature is obtained from the intersection point of the 1/2 line between the two baselines and the calorimetric curve.

A solid dispersion of the present invention exhibits good dissolution of the triazole compound which can be represented by formula (I). "To exhibit good dissolution" signifies exhibiting dissolution which maintains or produces a super-saturated state of the triazole compound which can be represented by formula (I), for example exhibiting dissolution such that a super-saturated solution with a solution concentration which is higher than the solubility by 50% or more can be obtained when a dissolution test is conducted by means of the dissolution test method (paddle method) described in the Fifteenth Edition of the Japanese Pharmacopoeia.

A solid dispersion of the present invention which has been subjected to pulverization can be used as a pharmaceutical composition as it is or after being mixed with other optional components which can be used in the field of pharmaceutical preparations.

No particular limitation in imposed upon the other components provided that they are pharmaceutically acceptable and pharmacologically acceptable. Examples include excipients, binding agents, disintegrants, acidulants, foaming agents, artificial sweeteners, flavors, lubricants, coloring agents, stabilizers, buffering agents, antioxidants, surfactants, coating agents and the like.

Furthermore, a pharmaceutical preparation of the present invention can take various forms, such as tablets, capsules, powders, granules and the like, and these can be produced by means of known methods. For example, pharmaceutical preparations (powders, fine granules, granules, tablets, capsules, and the like) can be prepared by following formulation processes such as mixing processes, granulation processes, tablet stamping processes, capsule filling processes, coating processes and the like.
As shown in Japanese Domestic Patent 2004-521964, a triazole compound which can be represented by formula (I) is a compound which has an analgesic action, cyclooxygenase inhibitory activity, inhibitory activity against platelet aggregation, therapeutic action for dementia including Alzheimer's disease and the like. Hence a pharmaceutical composition of the present invention which includes a triazole compound which can be represented by formula (I) can be used for the treatment and prevention of conditions such as inflammatory symptoms, pain, dementia including Alzheimer's disease, immunological diseases and the like. The pharmaceutical compositions of the present invention can be used in the form of pharmaceutical preparations which are suitable for oral, parenteral, or topical use (including but not limited to formulations suitable for delivery via a transdermal patch).

The amount of triazole compound which can be represented by formula (I) included in a pharmaceutical composition of the present invention differs according to the form of the preparation and the mode of administration but is, for example, from 0.5 to 50 mass%. The therapeutically effective dosage of a triazole compound which can be represented by formula (I) varies according to the age and symptoms of the individual patient, but an average dosage at one time of about 0.01 mg, 0.1 mg, 1 mg, 3 mg, 10 mg, 25 mg, 50 mg, 75 mg, 100 mg, 150 mg, 200 mg, 250 mg, 500 mg and 1000 mg of the triazole compound which can be represented by formula (I) can be effective for the treatment of the abovementioned conditions. Said dose can be administered once per day, twice per day or three times per day, or it can be administered by means of a continuous release preparation. Generally the dose per day will range from an amount of 0.01 mg to about 1,000 mg, and in some embodiments the dose per day will range from about 0.1 mg to about 750 mg, or from about 1 mg to about 500 mg.

### Illustrative Examples

The invention is described in more detail below by means of examples and comparative examples, but the invention is not limited by these examples.

The Compound A was produced according to the method described in Example 5 of the Japanese Domestic Patent (Kohyo) 2004-521964. Furthermore, the numbers in the column for each component in each table are the mass proportions of the component which had been used.

### Production of Solid Dispersions:

### Example 1

Compound A (300 g) and 300 g of polyvinylpyrrolidone (product name: PVP K30, produced by the BASF Co., same hereinafter) were mixed and then subjected to a melting process using a twin-screw extruder (product name: KEX-25, produced by the Kurimoto Tekkojo Co., same hereinafter) which had been set to a barrel temperature of 150°C and a screw rotation rate of 200 rpm and a solid dispersion was produced.

### Example 2

A solid dispersion was produced in the same way as in Example 1 except that copolyvidone (product name: Kollidon (registered trade mark) VA64, produced by the BASF Co., same hereinafter) was used instead of the polyvinylpyrrolidone.

### Comparative Examples 1 to 6

Solid dispersions were produced in the same way as in Example 1, except that hydroxypropyl methylcellulose (product name: TC-5E or TC-5R, both produced by the Shin-Etsu Chemical Co.), methylcellulose (product name: Metolose 60SH, produced by the Shin-Etsu Chemical Co.), aminoalkyl methacrylate copolymer EPO (product name: Eudragit E, produced by the Evonik Degussa Japan Co.), methacrylic acid copolymer L (product name: Eudragit L100, produced by the Evonik Degussa Japan Co.) or hydroxypropyl cellulose (product name: HPC-L, produced by the Nippon Soda Co.) was used instead of the polyvinylpyrrolidone.

### Example 3 to 6

Solid dispersions were produced on the basis of the formulations shown in Table 2.

PVP (600 g or 900 g) or copolyvidone (600 g or 900 g) was mixed with 300 g of Compound A and then solid dispersions were produced in the same way as in Example 1.

### Comparative Examples 7 and 8

HPMC (600 g or 900 g) was mixed with 300 g of Compound A and solid dispersions were produced in the same way as in Example 1.

### Examples 7 to 9

Compound A (300 g), 300 g of HPMC (product name: TC-5R, produced by the Shin-Etsu Chemical Co.) and 300 g, 450 g or 600 g of PVP were mixed and then subjected to a melting process using a twin-screw extruder which was set to a barrel temperature of 150°C and a screw rotation rate of 200 rpm and in each case a solid dispersion was produced.

### Example 10

A solid dispersion was produced in the same way as in Examples 7 to 9 except that copolyvidone (450 g) was used instead of the polyvinylpyrrolidone.

### Comparative Example 9

Compound A (1 part by mass), 1 part by mass of HPMC and 1 part by mass of PVP were dissolved in a mixed acetone: water (7:3) solvent and then a solid dispersion was produced by means of the spray drying method.

### Evaluation

### Pulverization:

With each Example and Comparative Example the solid dispersion which was discharged from the twin-screw extruder was pulverized using a pin mill (CoroPlex 160Z: produced by the Hosokawa Micron Co.) to produce solid dispersion particles. The solid dispersions of the examples were easily pulverized (for example, in the case of the solid dispersion of Example 7 the mass of pulverized material obtained was 97 mass% with respect to the weight before pulverization), but the solid dispersions of the comparative examples were difficult to pulverize and there was a problem in respect of productivity.

### Crystallinity:

Particles of the solid dispersions of Examples 3 to 10 and Comparative Examples 7 to 9 were taken in stoppered plastic containers and stored for one month under conditions of 40°C and 75% humidity after which the crystallinity of Compound A contained in the dispersion was evaluated by means of powder X-ray diffraction measurements (RINT TTRIII, produced by the Rigaku Co.). Furthermore, the crystallinity of the Compound A in the solid dispersion of each of the examples and comparative examples before storage was evaluated in the same way. A sharp diffraction peak pattern was observed in the X-ray diffraction measurements in those cases where the Compound A was in a crystalline form.

The results are shown in Tables 1 to 3. The term "amorphous" in the tables indicates that the Compound A was in an amorphous state and the term "crystalline" indicates that the Compound A was in a crystalline form.

Furthermore, measurement diagrams of the X-ray diffraction measurements and differential scanning calorimetry measurements (differential scanning calorimeter produced by the Seiko Co., rate of temperature increase 5°C/min) obtained after storage with Example 7 are shown in Figure 1 and Figure 2. Moreover, in Figure 1 the incident angle 2θ (°) is shown on the abscissa and the diffraction intensity is shown on the ordinate, and in Figure 2 the temperature (°C) is shown on the abscissa and the heat flow (mW) is shown on the ordinate. A broad pattern (halo pattern) with no clear diffraction peaks was obtained in the X-ray diffraction measurements (Figure 1) and no peak was observed in DSC (Fig. 2) and so it was ascertained that the Compound A was in an amorphous form even after storage. Moreover, the measurement diagrams of the X-ray diffraction measurements and differential scanning calorimetry measurements before storage were essentially the same as Figure 1 and Figure 2 and the Compound A was in an amorphous form.

### Dissolution:

Particles of the solid dispersion of each example and comparative example were weighed out and, using 900 mL of purified water for a test solution, testing was carried out at 50 rpm in accordance with the second dissolution test method of the Fifteenth Edition of the Japanese Pharmacopoeia. After the test started, 5 mL of the dissolution solution was collected periodically and filtered using a membrane filter and the concentration of Compound A dissolved in the filtrate was measured using the HPLC method. The measured concentrations after 60 minutes are shown in Table 2 and Table 3.

### Glass Transition Point (Tg)

Particles of the solid dispersion (about 10 mg) of each example and comparative example were weighed out on an aluminum pan and calorimetric measurements were made at temperatures between 0°C to 150°C with a rate of temperature increase of 5°C per minute. Heat was absorbed or generated at the glass transition point in the course of heating the sample and, as a result, the baseline of the calorimetric curve shifted downward. Extended lines were drawn for the two baselines, namely the original baseline and the shifted baseline, and the glass transition point was obtained from the intersection point of the 1/2 line between the two baselines and the calorimetric curve. The results obtained are shown in Tables 1 to 3.

**Table 1**

| | Example 1 | Example 2 | Comparative Example 1 | Comparative Example 2 | Comparative Example 3 | Comparative Example 4 | Comparative Example 5 | Comparative Example 6 |
|---|---|---|---|---|---|---|---|---|
| Polymeric Carrier | PVP | Copolyvidone | TC-5E | TC-5R | Metolose 60SH | Eudragit E | Eudragit L | HPC-L |
| Pulverization | Easy | Easy | Difficult | Difficult | Difficult | Difficult | Difficult | Difficult |
| Crystallinity (before storage) | Amorphous | Amorphous | Crystalline | Amorphous | Amorphous | Amorphous | Crystalline | Crystalline |
| Tg (°C) | 45 | 40 | - | 12 | 12 | 6 | - | - |

**Table 2**

| | Comparative Example 7 | Comparative Example 8 | Example 3 | Example 4 | Example 5 | Example 6 |
|---|---|---|---|---|---|---|
| Compound A | 1 | 1 | 1 | 1 | 1 | 1 |
| HPMC (TC-5R) | 2 | 3 | - | - | - | - |
| Copolyvidone | - | - | 2 | 3 | - | - |
| PVP | - | - | - | - | 2 | 3 |
| Pulverization | Difficult | Difficult | Easy | Easy | Easy | Easy |
| Crystallinity (before storage) | Amorphous | Amorphous | Amorphous | Amorphous | Amorphous | Amorphous |
| Crystallinity (after storage) | Crystalline | Crystalline | Amorphous | Amorphous | Amorphous | Amorphous |
| Dissolution (µg/mL) | 22.5 | 22.6 | 16.3 | 16.4 | 17.6 | 17.6 |
| TG (°C) | - | 36 | 52 | 68 | 73 | 88 |

**Table 3**

| | Example 7 | Example 8 | Example 9 | Example 10 | Comparative Example 9 |
|---|---|---|---|---|---|
| Compound A | 1 | 1 | 1 | 1 | 1 |
| HPMC (TC-5R) | 1 | 1 | 1 | 1 | 1 |
| Copolyvidone | - | - | - | 1.5 | - |
| PVP | 1 | 1.5 | 2 | - | 1 |
| Pulverization | Easy | Easy | Easy | Easy | - |
| Crystallinity (before storage) | Amorphous | Amorphous | Amorphous | Amorphous | Amorphous |
| Crystallinity (after storage) | Amorphous | Amorphous | Amorphous | Amorphous | Crystalline |
| Dissolution (µg/mL) | 22.5 | 22.0 | 21.6 | 22.2 | - |
| TG (°C) | 58 | 68 | 70 | 55 | 40 |

The solid dispersions which had a Tg of 40°C or above which had been produced by using copolyvidone or PVP for the polymeric carrier were easy to pulverize and were excellent in terms of handlability (Tables 1 and 2).

The solid dispersions which had been produced using two types of polymer, namely HPMC and copolyvidone or PVP, gave good results in all of the aspects of crystallinity, dissolution and pulverization (Table 3).

The solid dispersions which had been produced using only HPMC (Comparative Examples 7 and 8) had excellent dissolution properties but were difficult to pulverize and difficult to handle. However, solid dispersions which were easily pulverized were obtained when part of HPMC was replaced by copolyvidone or PVP (Examples 7 to 10). Hence, solid dispersions of the invention which were excellent in all of the aspects of crystallinity, dissolution and ease of handling could be produced by combining a polymer selected from PVP and copolyvidone with the HPMC.

The solid dispersions which had been produced by means of a spray drying process using HPMC and PVP had a low glass transition point when compared with the solid dispersions which had been produced by means of a melting process and they had lower stability (retention of the amorphous form during storage) (Example 7 and Comparative Example 9).

## Claims

1. A solid dispersion comprising a triazole compound represented by formula (I): 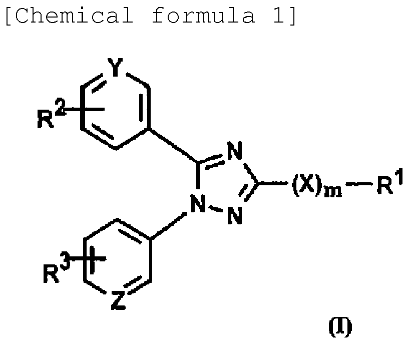 [in the formula, R¹ is a lower alkyl group optionally substituted with halogen, cyano, N,N-di(lower)alkylcarbamoyl, phenyl optionally substituted with halogen, or heterocyclic group,
a cyclo(lower)alkyl group,
a lower alkynyl group, or
an N,N-di(lower)alkylcarbamoyl group;
R² is a lower alkyl group, lower alkoxy group, cyano group or 1H-pyrrol-1-yl group;
R³ is a lower alkyl group, lower alkoxy group or cyano group;
X is O, S, SO or SO₂;
Y and Z are each CH or N;
m is 0 or 1];
and also comprising a polymeric carrier, the solid dispersion being non-crystalline and having a glass transition temperature of at least 40°C (e.g. 80°C).

2. The solid dispersion as claimed in claim 1, wherein the triazole compound is 3-methoxy-1,5-bis(4-methoxyphenyl)-1H-1,2,4-triazole.

3. The solid dispersion as claimed in claim 1 or 2, wherein the polymeric carrier is one or two types of carrier selected from the group consisting of polyvinylpyrrolidone and copolyvidone.

4. The solid dispersion as claimed in any one of claims 1 to 3, further comprising hydroxypropyl methylcellulose.

5. The solid dispersion as claimed in claim 4, wherein hydroxypropyl methylcellulose is compounded with a polymeric carrier.

6. The solid dispersion as claimed in claim 1, which contains 1 - 10 parts by mass of polymeric carrier with respect to 1 part by mass of triazole compound represented by formula (I).

7. The solid dispersion as claimed in claim 4 or 5, which contains 1 - 3 parts by mass of hydroxypropyl methylcellulose with respect to 1 part by mass of triazole compound represented by formula (I).

8. The solid dispersion as claimed in any one of claims 1 to 7, which is produced by means of a melting process.

9. A pharmaceutical composition comprising the solid dispersion as claimed in any one of claims 1 to 8.

10. A method for producing the solid dispersion as claimed in claim 1, comprising:
a step of mixing a triazole compound represented by formula (I) and a polymeric carrier to prepare a mixture; and
a step of melting the mixture.
